# EUROPEAN PATENT APPLICATION

(11) **EP 0 626 170 A2**
(43) Date of publication of application: **30.11.1994**
(21) Application number: 94810273.6
(22) Date of filing: 09.05.1994
(51) Int. Cl.: A61K 9/16, C07K 7/06, C07K 15/04

(54) **Stabilisation of pharmacologically active compounds in sustained release compositions**

(30) Priority: 10.05.1993 GB 9309543; 10.05.1993 GB 9309550
(71) Applicant: SANDOZ LTD., CH-4002 Basel (CH); SANDOZ-PATENT-GMBH, D-79539 Lörrach (DE); SANDOZ ERFINDUNGEN VERWALTUNGSGESELLSCHAFT M.B.H., A-1235 Vienna (AT)
(72) Inventor: Bodmer, David, CH-5313 Klingnau (CH); Gengenbacher, Thomas, D-79108 Freiburg-Hochdorf (DE); Esparza, Inmaculada, CH-4051 Basle (CH)

(57) **Abstract**

A pharmaceutical composition comprising as the active agent a peptide in association with embonic acid or ascorbic acid or a salt thereof, excluding
1) ACTH in association with embonic acid or acorbic acid or a salt thereof and
2) a LHRH active compound in association with ascorbic acid or a salt thereof,
   in a matrix of a polylactide, having a lactide / glycolide molar ratio of from 100:0 to 40:60, a Mw of from 10.000 to 200.000 and a polydispersity of from 1.7 to 3.0.

## Description

The invention relates to a pharmaceutical composition of a water soluble pharmacologically active peptide in a matrix of a biodegradable polyester having an excellent agent release profile and stability.

The invention provides a pharmaceutical composition comprising as an active agent a peptide e.g. a protein in association with embonic acid or ascorbic acid or a salt thereof, excluding
1) ACTH in association with embonic acid or ascorbic acid or a salt thereof and
2) a LHRH active compound in association with ascorbic acid or a salt thereof,

in a matrix of a polylactide, having a lactide/glycolide molar ratio of from 100:0 to 40:60, a Mw of from 10.000 to 200.000 and a polydispersity of from 1.7 to 3.0.

The LHRH active compounds comprise LHRH agonists and antagonists and their analogues and salts thereof.

The peptide may be a protein.

Usable peptides are such having a molecular weight of 1 to 300 k Dalton, e.g. enzymes and blood factors (Mw 20 - 300 k Da), immunizing antigens and antibodies (Mw up to 200 k Da), cytokines (Mw 10 - 30 k Da) and peptides having a molecular weight of 1 to 10 k Da. The most usable peptides have a molecular weight range of 1 more preferably 1.5 to 150 k Da.

According to the PCT-patent application WO 93/17668 ACTH controlled release sytems in polymers are described containing excipients which are polymer degradation enhancers, e.g. organic acids like benzoic acid and ascorbic acid (page 14, lines 4-13).

The US-patent 4,675,189 describes e.g. organic acids and their salts, e.g. ascorbic acid to be used as polymer hydrolysis modifying agents in LHRH active controlled release systems in polymers, e.g. polylactides (Column 11, lines 5-19).

It could not be expected, that these type of compounds may improve the stability of pharmacologically active peptides and proteins in polymers, especially polylactides.

According to the present invention it has been found that embonic acid and ascorbic acid behave as stabilisators of peptides, e.g. proteins in polylactides, especially those peptides not excluded in the combination as mentioned before.

The invention especially provides a pharmaceutical composition comprising such a peptide in a matrix of the polylactide mentioned above, the composition showing, after manufacture thereof, an increase of at most 0.5% in active agent degradation products resulting in a total degradation product content of at most 2.5% and the active agent being in association with embonic acid or ascorbic acid or a salt thereof and the composition showing in an aqueous medium an active agent release which is continuous during a period of at least 2 weeks and which amounts within the first 24 hours to at most 15% (e.g. at most 10%, preferably at most 6%), of weight of active agent, released.

The composition preferably has measured from the beginning of manufacture to just after irradiation for sterilization with e.g. 25 kGy, an increase of degradation products of at most 2.0%, preferably at most 1%, especially at most 0.5%.

In particular, such composition is preferred having measured from the beginning of manufacture over irradiation to just after storage during 3 months at 25°C a degradation product increase of at most 2.5%, preferably at most 1.5%.

The composition contains the peptide or protein in the association with embonic acid.

Alternatively the peptide or protein is in an association with a salt of the acid. e.g. an alkali salt, especially the sodium salt.

The peptide or protein is also considered to be in the association with the acids, if the peptide or protein is present as a salt with the acids mentioned above, especially the peptide or protein as the pamoate salt.

The peptide of most interest is CH₃-CO-D-2-Nal-DPhe(p-CI)-DTrp-Ser-Tyr-DLys(CH₂-CHOH-CH₂OH)--Leu-Lys(CH₂--CHOH-CH₂OH)-Pro-DAla-NH₂. It is called compound A.

This compound is a water soluble peptide and is disclosed in PCT-Application WO 89/09786 in Example 1.

The compound in free form or in the form of pharmaceutically acceptable salts and complexes exhibits valuable pharmacological properties as indicated in animal tests and is therefore indicated for therapy.

Compound A is a LHRH antagonist and inhibits luteinizing hormone secretion, e.g. as indicated by an inhibition of ovulation in animals. This test is effected according to M. Marko and E. Flückiger, Experientia 30, 1174 - 1176 (1974).

The LHRH antagonist is active in this test when administered at a dosage in a range from about 0.0005 to about 10 mg/kg s.c.

The inhibiting effect on luteneizing hormone secretion of the LHRH antagonist can also be tested in vitro:

Methods in Enzymology 37, 82 - 93 (1975) as has been described previously (M. Marko and D. Römer: Life Sciences, 33, 233 - 240 (1983).

LHRH antagonist peptides show antagonistic effects against LHRH in a concentration below 10^{-7 M.}

The LHRH antagonist compound A is accordingly useful in the treatment of conditions making a suppression of gonadotropic secretion medically desirable such as pubertas praecox, mammary cancer, prostatic hypertrophy and prostatic cancer, endometriosis, and gonadotropin secreting pituitary tumors, and for suppressing ovulation in female and spermatogenesis in male.

Compound A may be administered in free form or in pharmaceutically acceptable salt form or complexes. The salts and complexes exhibit the same order of activity as the free compound. The compound may be administered by any conventional route, for example parenterally, intramuscularly or subcutaneously e.g. in form of injectable suspensions for which the composition of the invention is a preferred one.

The polyester in the composition is preferably a polylactide, which is biodegradable.

Its degradation time in the body may easily be fixed within wide limits, e.g. from one or more days, e.g. one or two weeks to one or more months, e.g. one month, by regulation of the lactide/glycolide molar ratio between 100:0 and 40:60 and by the molecular weight.

The main molecular weight M_{w} is in the range of from about 10,000 to 200,000 preferably 25,000 to 100,000, especially 35,000 to 60,000 and polydispersity e.g. of from 1.7 to 3.0, e.g. 2.0 to 2.5.

Preferably the polymer has a polymer lactide: glycolide molar ratio of from 60:40 to 40:60.

The most preferred polylactide-co-glycolide is a star polymer, e.g. polylactide-co-glycolide glucose, which has been described in the UK Patent GB 2 145 422 B.

The intrinsic viscosities of star polymers of M_{w} 35,000 and M_{w} 60,000 are 0.36 and 0.51 dl/g, respectively in chloroform. A star polymer having a M_{w} 52,000 has a viscosity of 0.475 dl/g in chloroform.

The composition of the invention is preferably in the form of microparticles or of an implant, which may be produced by manners known per se. The preparation of the microparticles is e.g. described in the British Patent GB 2 145 422 B or in the British patent application GB 2 234 896 A.

The agent's loading percentage is preferably between 0.01 and 10%, more preferably below 8%, especially to 6%, based on the weight of polylactide.

The peptide, e.g. the protein has in association with especially pamoic acid or a salt thereof an excellent stability during the preparation of the composition, its irradiation and its storage, notwithstanding there are several harmful factors which threaten its chemical structure, e.g. destructive influences of the polymer polyester and further environmental influences.

The invention provides additionally a method of stabilizing Compound A in the biodegradable polylactide which comprises bringing the active agent in association with embonic acid or ascorbic acid.

In yet a further aspect the invention provides use of embonic acid or ascorbic acid to stabilize Compound A in the biodegradable polylactide.

The invention provides accordingly compound A in embonic acid or ascorbic acid salt form and in another aspect such a salt in a matrix of a biodegradable polylactide.

The invention additionally relates to a pharmaceutical composition of tetanus toxoid active agent in a matrix of the biodegradable polylactide mentioned before and having a good stability.

The invention provides, in one aspect, the active agent in the composition in association with embonic acid or ascorbic acid or a salt thereof as the stabilizer.

In another aspect the invention provides a method of stabilizing a tetanus toxoid in the biodegradable polylactide which comprises bringing the tetanus toxoid in association with embonic acid or ascorbic acid or a salt thereof.

In yet a further aspect the invention provides use of embonic acid or ascorbic acid or a salt thereof to stabilize tetanus toxoid in the biodegradable polylactide.

Tetanus toxoid is an immunizing antigen protecting against the adverse effects of infection by tetanus organisms. It is prepared from the toxin produced by the growth of a highly toxigenic strain of Clostridium tetani in a suitable medium. The supernatant fluid, which contains the toxin, is separated from the organisms; the toxin so separated from the organisms is detoxified and purified.

The toxoid is a very good antigen and even plain toxoid (a toxoid without the addition of an adjuvant) given in adequate dosage can be relied upon for protection when used for primary immunization. It is common, however, for the toxoid to be adsorbed on to an adjuvant (usually aluminium hydroxide or phosphate).

The toxin is a highly toxic protein with a molecular weight close to 150,000. It is synthesized as a single polypeptide chain. This toxin can be extracted from the washed bacteria, before it has diffused into the culture medium, by treatment with neutral hypertonic solutions. The extracted toxin is designated as intracellular toxin. However, when the toxin is discharged into the culture medium at the time of cell autolysis, it is cleaved by the action of proteases present in the culture medium to yield the nicked toxin, or extracelluloar toxin, which is the one used in the vaccine preparation. This results in an aminoterminal light chain of around 52,000 daltons and a carboxyterminal heavy chain of 98,000 daltons (fragments A and B-C respectively in Fig. A).

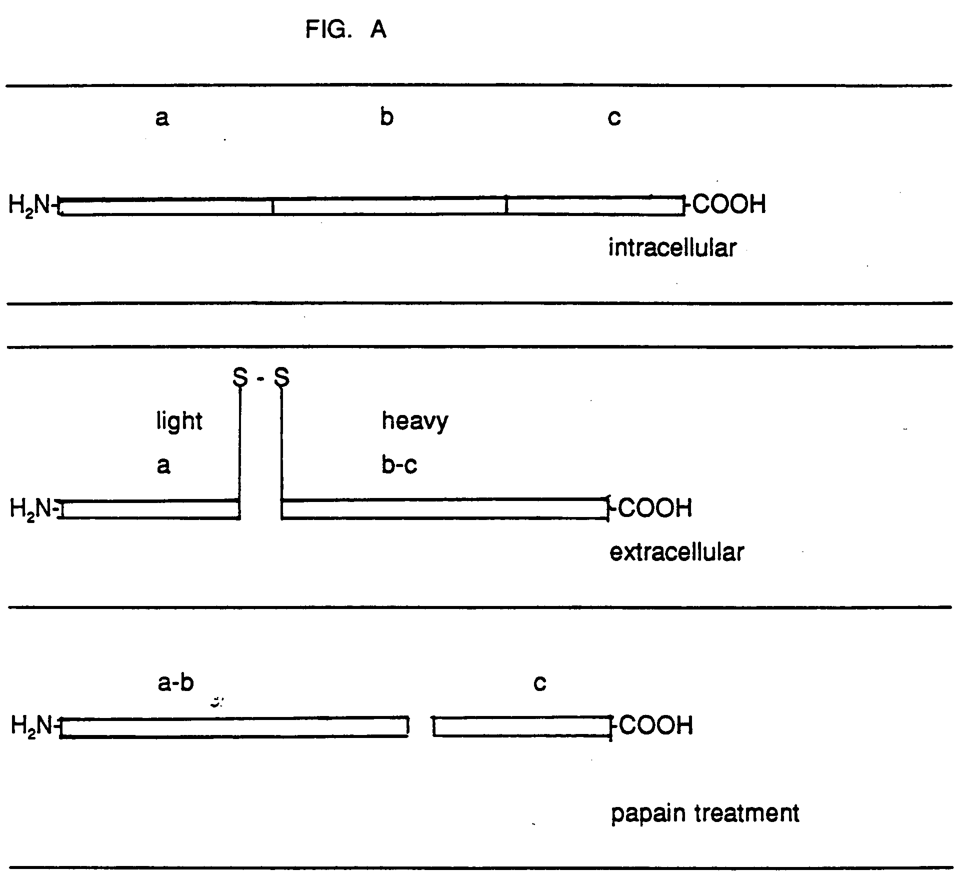

Light and heavy chains are held together by a disulphide bridge. Treatment of the toxin with papain cleaves the protein in an aminoterminal fragment A-B of 99,000 daltons and a carboxyterminal fragment C of 51,000 daltons.

The toxin can be neutralized by monoclonal antibodies binding fragment A, B or C, suggesting that in theory any of the 3 non-toxic fragments could be used as a vaccine. Fragments A, B and C have been successfully used to immunize mice against tetanus.

To produce the conventional tetanus vaccine, C. tetani is grown in a semisynthetic medium in a fermentor for about one week, until the bacteria lyse and release tetanus toxic into the supernatant.

The culture is then filtered and the filtrate containing the toxin is detoxified by adding formaldehyde to a final concentration of 0.5%. The pH is adjusted to 7.6 and the supernatant is then stored at 37°C for 4 weeks to allow the complete detoxification of tetatus toxin. During the detoxification process, formaldehyde reacts with the toxin molecules, peptones and other proteins present in the medium.

The final result of the formaldehyde treatment of tetanus toxins involves a cross-linkage between an ε-aminogroup of lysine and: (a) a second amino group, (b) an histidine and (c) a thyrosine or a tryptophan, through a stable methylene bridge (-CH₂-). These products are present in the acid hydrolysates of toxcoids and can be easily identified by aminoacid analysis.
Obviously, these reactions can occur (1) between amino acids of the same toxin molecule, resulting in internal cross-linking of the protein; (2) between two toxin molecules, resulting in dimerization or (3) between a small peptide present in the medium and a toxin molecule.

Following detoxification, the crude toxoids are concentrated by ultrafiltration and may then be purified by fractionation with ammonium sulphate, dialysis, gel filtration, chromatography on DEAE-cellulose or a combination of the above methods. Since the detoxification process has created a population of molecules which are extremely heterogeneous, none of the above purification methods can give a pure product and usually their purity is in the range of 30-70%. The toxoids are then tested for potency, toxicity, sterility, and reversibility and stored at 4°C. For vaccine preparation they are adsorbed onto aluminium hydroxide or calcium phosphate.

The composition contains the toxoid agent in association with, e.g. mixed with embonic acid or ascorbic acid.

Alternatively the active agent is in association with the salts of the acids, e.g. the alkali salts, especially the sodium salts.

The active agent is also considered to be in association with the acids, if the toxoid is present as a salt with the acids mentioned-above, especially the tetanus toxoid as the pamoate salt. Alternatively, the active agent is in association with ascorbic acid or a salt thereof.

The matrix in the composition is a polylactide, which is mentioned above.

Its lactide: glycolide molar ratio is from 100:0 and preferably from 90:10 especially 60:40 to 40:60, e.g. 85:15 and 55:45. In order to obtain a pulsatile release of the agent, e.g. an initial peak for priming (induction of immune response) and, later, a second and optionally a third peak for boosting after one or two months to more months, e.g. one and six months, two or three types of microsphere preparations each composed of the toxoid agent, the mentioned acids or salts thereof and one of 2 or 3 different polyesters may be mixed and used as a single vaccination formulation. The preferred molar ratios of lactide:glycolide would be 55:45 for an immediate release, 85:15 for an intermediate release and 100:0 for a delayed release.

The agent's loading percentage is preferably between 0.01 and 10%, more preferably up to 1%, especially up to 0.7% e.g. 0.5% related to the weight of the polylactide.

An indicated monthly dose for larger mammals, e.g. humans is 20 mg of a 0.5% loaded composition.

The tetanus toxoid has in association with especially pamoic acid or ascorbic acid or their salts thereof, an excellent stability during the preparation of the composition, its irradiation and storage, notwithstanding there are several harmful factors, which threaten the chemical structure, e.g. destructive influence of the polymer polyester and further environmental influences.

The tetanus toxoid stability is determined by using a very sensitive method, e.g. enzyme-linked immuno sorbent assay (ELISA), measuring the toxoid activity.

The formulations are useful for the known indications of the particular active agents incorporated therein.

The exact amounts of active agents and of the formulations to be administered depend on a number of factors, e.g. the conditions to be treated, the desired durations of treatment and the rates of release of the active agents.

For example, the amount of the active agents required and the release rates thereof may be determined on the basis of known in vitro or in vivo techniques, determining how long a particular active agent concentration in the blood plasma remains at an acceptable level for a therapeutic effect.

Examples of doses for the preferred compounds are:
a) for use in the treatment of conditions making a suppression of gondotropic secretion medically desirable such as pubertas praecox, mammary cancer, prostatic hypertrophy and prostatic cancer, endometriosis, and gonadostropin secreting pituitary tumors, and for suppressing ovulation in female and spermatogenesis in male, with compound A, for example an i.m. depot formulation may be produced which daily provides 0.3 mg to about 60 mg of compound A.
   The compositions of the invention containing compound A are designated to release this daily dose over 1 -2 months. A typical example is an i.m. depot formulation containing 6 mg of compound A and releasing daily 100 microgram of compound A over a period of 60 days.
   In the compositions compound A may present in free form or in pharmaceutically acceptable salt form or complexes. The salts and complexes exhibit the same order of activity as the free compound. The composition may be administered by any conventional route, for example parenterally, intramuscularly or subcutaneously e.g. in form of injectable suspensions. The compositions of the invention can develop serum levels, which due to the excellent stability and release properties, are better suitable for therapeutic purposes than conventional preparations which have to be administered several times a day, when not in sustained release form. They admit a satisfactory release in wide limits, e.g. from one or more days, e.g. one or two weeks to one or more months, e.g. one month.
b) For use in the treatment of tetanus a tetanus toxoid depot formulation may be produced in the form of microparticles containing 0.5% of tetanus toxoid. Satisfactory results in mice are obtained after subcutaneous injection of one or two mg of microparticles.
   In larger mammals, e.g. humans an intramuscular administration of microparticles at a dosage in the order of 100 mg is appropriate.

### EXAMPLE 1:

### Preparation of the pamoate salt of compound A

a) preparation of sodium embonate
   3.88 g of embonic acid are suspended in water. While stirring, the suspension was dissolved by adding 20 ml 1N NaOH, after which the volume of the solution was adjusted to 100 ml (0.1 molar),
b) preparation of compound A pamoate
   1.0 g of lyophilized compound A acetate was dissolved in 80 ml of water and 13 ml of 0.1 molar sodium pamoate was added while stirring the solution.
   The mixture was centrifugated. The residue was washed several times with water and then in water finely suspended and lyophilized.
   The lyophilized product was characterized with NMR and it was found a 1:1 molar ratio between compound A and embonic acid.
c) preparation of compound A phosphate
   After cleavage from the peptide resin and purification via HPLC on a RP-18 column as described in the PCT Application WO 89/09786 (acetonitrile gradient - 2% H₃PO₄ as eluent) pure fractions were pooled and treated with such an amount of an ion exchanger free base (e.g. BioRad AG-4,X₄) until the pH of the solution reached about 7.3.
   The ion exchanger was filtered off and washed. The volume of the filtrate was reduced in vacu, and then liyphilized, giving the monophosphate.
   Found: 0.8 - 1.2 equivalents of phosphate by microanalysis.
   [α]²⁰_{D} = -37° (c = 1.0 in 95% of AcOH).

### EXAMPLE 2:

### Preparation of microparticles containing the pamoate salt of compound A

The polymer used was a branched poly (DL-lactide-co-glycolide-D-glucose), [DL-PLG-GLU], with a molecular weight of approx. 55'000 and a molar ratio of lactide to glycolide of 55:45.

The polymer was prepared according to the method described in the british patent GB 2.145.422 B.

Microparticles were produced by a modified triple-emulsion technique. Compound A pamoate salt was dispersed in a small amount of water (14 mg pamoate salt in 0.1 ml) and the dispersion was intensively homogenized using an Ultra-Turrax T25 (Janke & Kunkel IKA-Werk) into a solution (10-25%) of DL-PLG-GLU in methylene chloride (emulsion process). Alternatively, compound A pamoate salt was directly dispersed into the solution of polymer in methylene chloride (suspension process). The resulting primary water/oil (W/O) emulsion or suspension (S/O) was then intensively mixed with the external phase (in a ratio of 1:40), a gelatin containing aqueous solution. After stirring for one hour in order to evaporate the methylene chloride, the generated microspheres were collected by filtration, washed with water and dried under high vacuum at room temperature for 1 - 3 days. Storage was effected at 5°C. Sterilization was effected by γ-irridation (25 kGy required) by using nitrogen as a protecting gas.

### Microparticle characterization

Compound A content and purity of the microparticles was determined by HPLC (RP-18 columns, acetonitril - tetramethyl ammonium hydroxide/water gradient) after extraction of the peptide from the microspheres using tetrahydrofuran acetate buffer, pH 4.2 (1:1).

Residual solvents: Methlyene chloride was determined by gas chromatrography (GC), water by Karl-Fischer titration.

In vivo characterization was performed in male rats. Microspheres containing compound A phosphate and pamoate were given s.c. (10 mg/kg) and serum levels of the compound and basal testosteron levels were measured.

### RESULTS AND DISCUSSIONS (2 batches of inventive composition)

Compound A pamoate salt was encapsulated in good yields (> 80%) into DL-PLG-GLU. Spherical particles with mean diameters of approx. 40 µm were obtained. Good flowability and applicability by syringes was observed.

### Encapsulation efficiency

At a theoretical drug loading of 6% the encapsulation efficiency was > 80%.

### In vivo testing

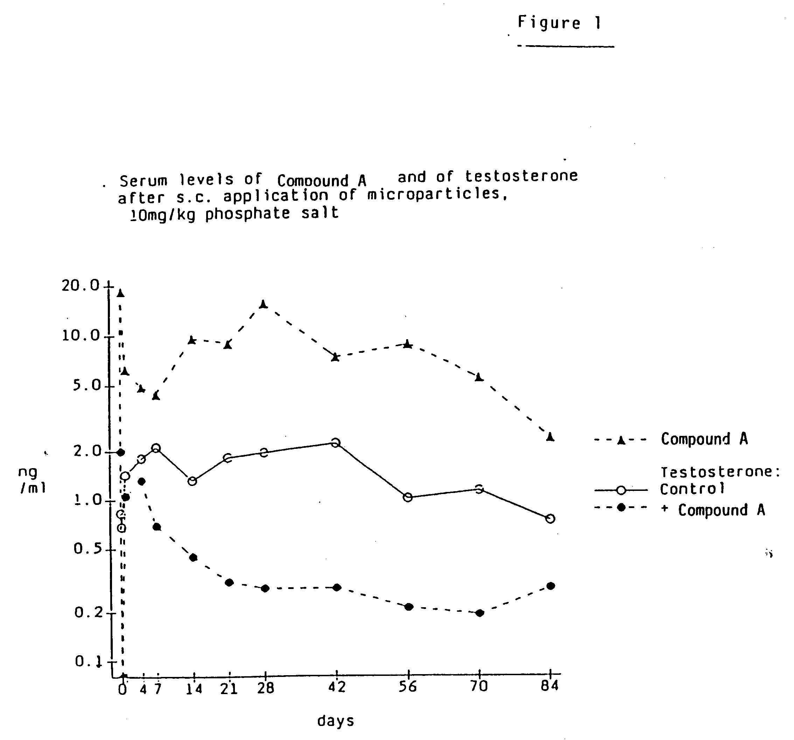

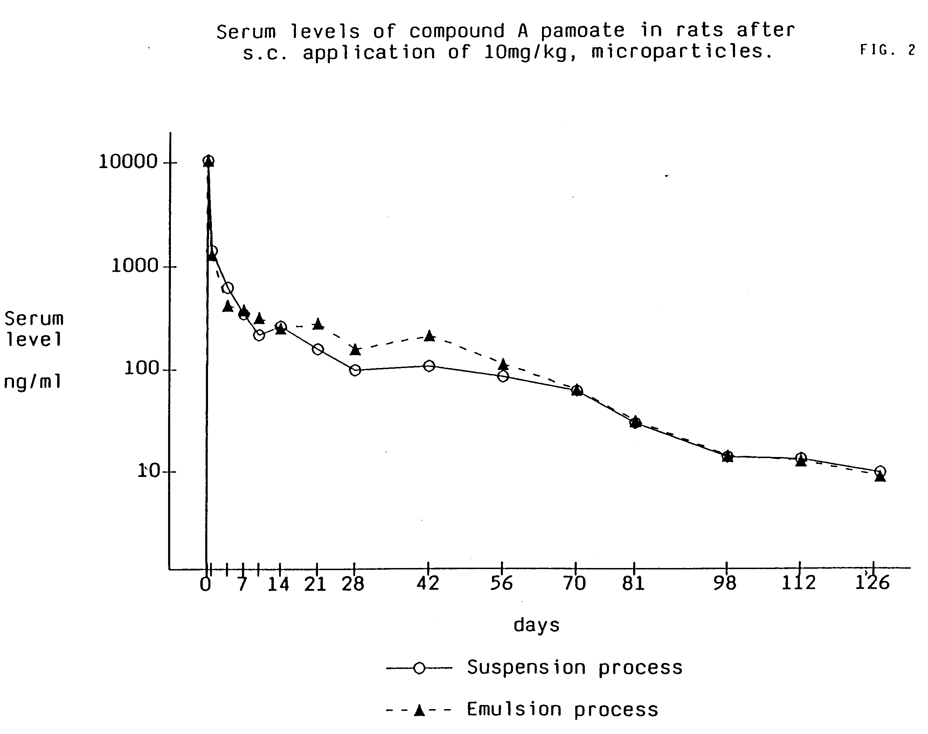

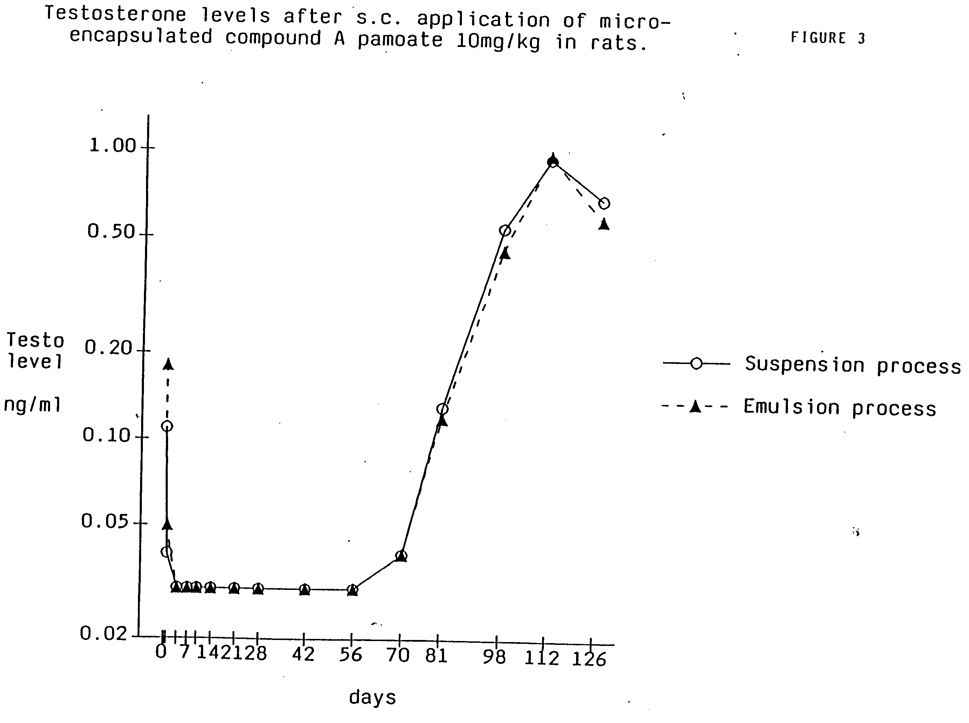

Results obtained in in vivo testing in rats with 2 batches of compound A phosphate microspheres are illustrated in Fig. 1, those with compound A pamoate microspheres in Fig. 2 + 3. The vehicle for administration is given in Table 1. The compound A plasma levels were sufficiently high-to inhibit the secretion of testosteron during 70 to 81 days. (See Fig. 3). Comparance of the release of the water soluble phosphate salt in Fig. 1 and of the pamoate salt in Fig. 2 indicates a far much higher serum level for the pamoate salt and a more pronounced drug burst.

The sterile formulations exhibited a biological effect in rats during at least two months, i.e. sufficient high plasma levels for total inhibition of basal testosteron (see Fig. 3).

### Stability

The generation of degradation products of compound A during the manufacture process, γ-irradiation and subsequent strorage at various temperatures is represented in Table 2. Potential stability problems arose with the acetate and the phosphate salts of compound A: during manufacture, sterilization and 1 month storage (5°C) 5 - 10% degradation occurred. These problems could be circumvented by the use of the pamoate. After manufacture, sterilization and three months of storage even at 25°C there was an increase of degradation products of less than 1.5%, more particularly of less than 0.5% after manufacture, less than 1.0% from the beginning of manufacture to just after irradiation and , inclusive of storage for 3 months at 25°C less than 1.5%.

### In vitro testing

The tests were carried out in pure water. Weighed amounts of 2 batches of microspheres were placed in 10 to 30 ml of dissolution medium in vials and shaken at 37°C. Samples were periodically drawn and peptide concentrations were assayed by HPLC analysis.

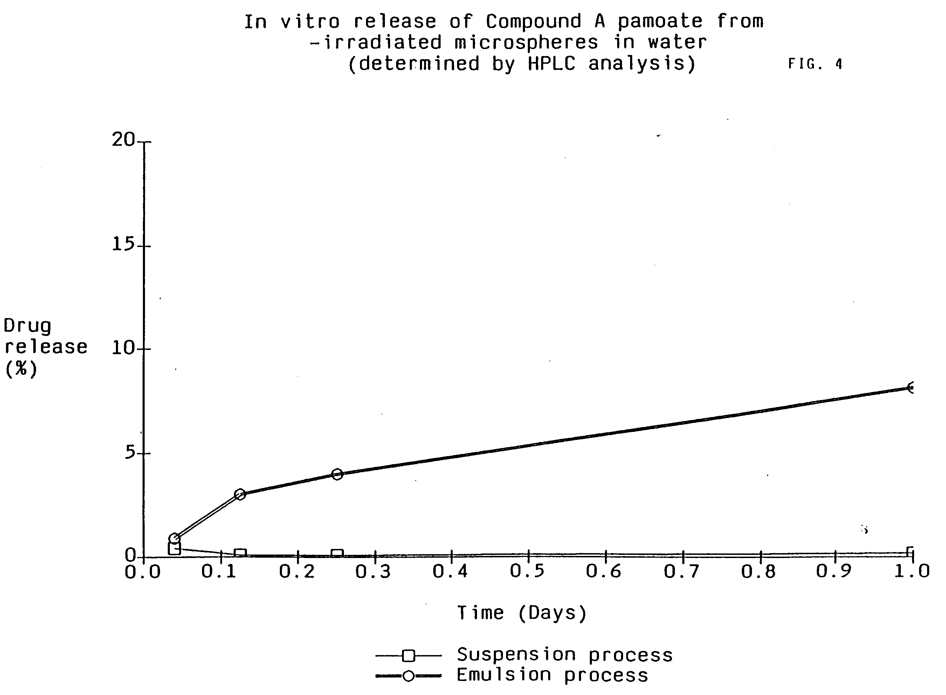

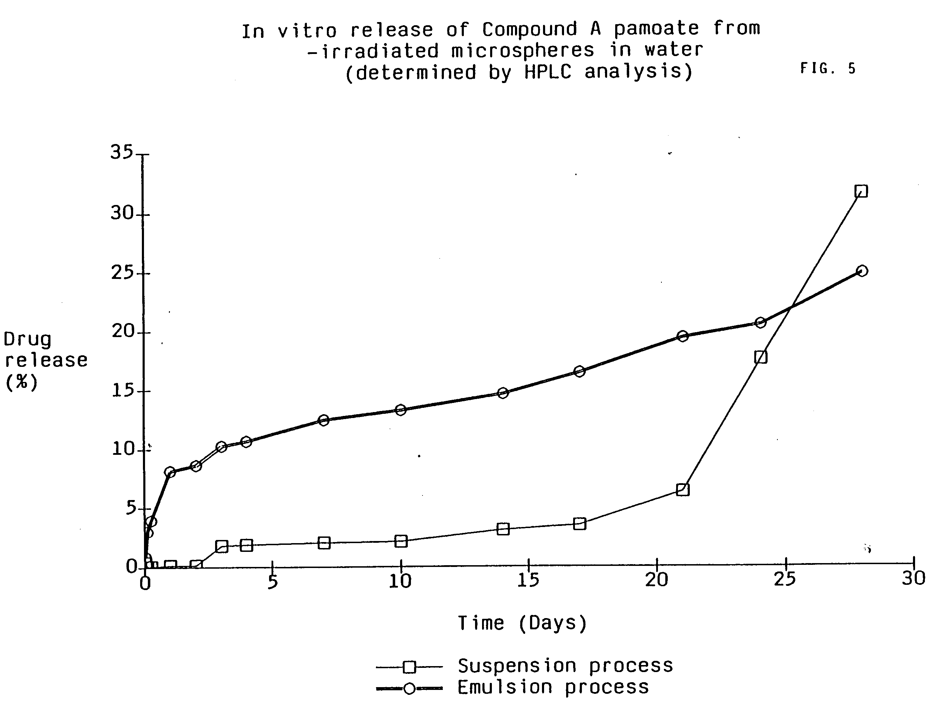

It is seen in Fig. 4 and 5, that the agent release does not exceed 15% in the first 24 hours and lasts at least 2 weeks, particularly at least 40 days, e.g. 2 months.

**TABLE 1**

| Vehicle for injection | |
|---|---|
| KH₂PO₄ | 3.60 mg/ml |
| Na₂HPO₄ | 5.68 mg/ml |
| Benzylalcohol | 10.0 mg/ml |
| Na-carboxymethylcellulose | 17.5 mg/ml |
| Polyethylene-polyoxypropylene-ether | 10.0 mg/ml |
| (Pluronic F 68) | |

**TABLE 2**

| Compound A Microspheres: Stability of Pilot Batches | | | | |
|---|---|---|---|---|
| Peptide Salt | Increase of degradation product (%) after | | | |
| | manufacture | man. + γ-irrad. | storage at | |
| | | | 5°C | 25°C |
| Compound A | | | 1 month | |
| acetate | 0.4 - 4.2 | 4.2 - 8.3 | + 0.6 | + 1.3 |
| Compound A | | | | |
| phosphate | 1.2 - 3.5 | 7.0 - 9.0 | + 0.7 | + 1.6 |
| Compound A | | | 3 months | |
| pamoate | 0.1 - 0.4 | 0.4 - 0.8 | + 0.4 | + 0.6 |

### EXAMPLE 3

a) Preparation of microparticles containing 0.5% of tetanus toxoid
   1 g of DL-polylactide-co-glycolide-D-glucose (DL-PLG/GLU), Mw = 54,975 (lactide/glycolide 85/15) was dissolved in 3.2 ml of methylene chloride with magnetic stirring followed by the addition of 5 mg of tetanus toxoid dissolved in 0.25 ml distilled water. The mixture was intensively mixed with an Ultra-Turax for one minute at 20,000 rpm (= inner W/O-phase).
   1 g of gelatin A was dissolved in 200 ml of 1/15M phosphate buffer (pH 7.4) at 50°C and the solution cooled down to 20°C (= outer W phase).
   The W/O- and the W-phases were intensively mixed. Thereby the inner W/O-phase was separated into small droplets which were dispersed homogenousely in the outer W-phase. The resulting triple emulsion was slowly stirred for 1 hour. Hereby the methylene chloride was evaporated and the microcapsules were hardened from the droplets of the inner phase. After sedimentation of the microparticles the supernatant was sucked off and the microparticles were recovered by vaccum filtration and rinsed with water to eliminate gelatin. Finally, microparticles were dried in a vacuum oven for 24 hour and sieved (0.180 mm mesh size) to obtain the final product.
b) Spray dried tetanus toxoid microparticles loaded with 2% of tetanus toxoid
   20 mg tetanus toxoid were carefully dissolved in 0.3 ml of distilled water. 1 g of DL-polylactide-co-glycolide-D-glucose (DL-PLG/GLU), Mw = 54.100 (lactide/glycolide 55/45) was dissolved in 20 ml of methylene chloride with magnetic stirring. Tetanus toxoid and polymer solution were combined and intensively mixed with an Ultra-Turax at 24,000 rpm for one minute, resulting in a W/O emulsion.
   This emulsion was spray dried with a spray dryer. Hereby the methylene chloride was evaporated and the microparticles were formed. Finally, the microparticles were dried in a vacuum over at room temperature for 48h and the resulting powder was sieved (0.180 mm mesh size) to obtain the final product.
c) Spray dried tetanus toxoid microparticles loaded with 0.5% of tetanus toixoid
   1 g of DL-polylactide-co-glycolide-D-glucose (DL-PLG/GLU), Mw = 54,100 (lactide/glycolide 55/45) was dissolved in 20 ml of methylene chloride with magnetic stirring. 5 mg of tetanus toxoid were added to the polymer solution and intensively dispersed with an Ultra Turax at 24,000 rpm for 1 min.
   The obtained microparticles were treated further in the manner as described under b).
d) Preparation of microparticles containing 0.5% of tetanus toxoid pamoate
   d.1 Preparation of tetanus toxoid pamoate
      100 mg of tetanus toxoid were dissolved in 6.85 ml of distilled water and 11.54 mg Na-embonate added. The mixtures was stirred for 1 hour and finally freeze dried for 48 hours. The resulting tetanus toxoid pamoate contained 89,7% of weight of tetanus toxoid.
   d.2 Preparation of tetanus toxoid pamoate microparticles
      1 g of DL-polylactide-co-glycolide-D-glucose (DL-PLG/GLU), Mw = 54,975 (lactide/glycolide 85/15) was dissolved in 3.2 ml of methylene chloride with magnetic stirring followed by the addition of 5.57 mg of tetanus toxoid pamoate in 0.25 ml distilled water.
      The mixture was intensively mixed, e.g. with an Ultra-Turax for one minute at 20,000 rpm ( = inner W/O-phase) and further treated in the manner, described under a).
e) Elisa method for the stability of tetanus toxoid microparticles
   20 mg of tetanus toxoid (TT) containing microprarticles were dissolved in 3 ml of dichloromethane and filtered on a 0.45 µm Ultipor^{R} membrane (Pall Corp.). After washing with 2 ml dichloromethane, the filter was extracted with 1 ml phosphate buffered saline (PBS) pH 7.2. The extract was serially diluted in PBS containing 1% bovine serum albumin on a human anti TT-IgG (Berna # 0604) coated microtiter plate (50 µl). After incubation overnight at 4°C, the plate was washed (3 x PBS 0.05% Tween) and incubated for 3 hours at 37°C with 50 µl murine anti-TT antisera (batch 207.92, 1/100). After washing, the plate was incubated with 50 µl anti-mouse IgG peroxidase conjugate (Bio-Rad, 1/500) for 1 hour at 37°C. After washing and addition of substrate solution (4mg/ml ABTS in 0.012% hydrogen peroxide/citric acid buffer pH 4.5), absorbances were read at 405 nm on an Elisa reader and plotted against the dilution factors.

### EXAMPLE 4

### Preparation of microparticles containing 0.5% tetanus toxoid and 0.5% Na-ascorbate

a) 1 g of DL-polylactide-co-glycolide-D-glucose (DL-PLG/GLU), Mw = 82,000 (lactide/glycolide 85/15) was dissolved in 6 ml of methylene chloride with magnetic stirring followed by the addition of 5 mg of tetanus toxoid and 5 mg of Na-ascorbate dissolved in 0.5 ml of distilled water.
   The mixture was intensively mixed, e.g. with an Ultra-Turax for one minute at 20,000 rpm (= inner W/O-phase) and further treated in the manner described in Example 3a. A part of the microparticles were irradiated with 25 kGy at 20°C.
b) The preparation of microspheres containing 0.5% of weight of tetanus toxoid and 0.5% of Na-ascorbate was repeated but instead of DL-PLG-GLU, M_{w} = 82,000; lactide/glycolide 85/15 a DL-PLG-GLU having a M_{w} = 59,000 Mₙ = 22,500, lactide/glycolide 55/45 was used.
   The microsphere samples were irradiated with 25 kGy at 20°C.
c) The preparation and irradiation under b) was repeated with the polymer L-polylactide, M_{w} = 46,400; Mₙ = 26,000, lactide/glycolide 100/0.

### Results

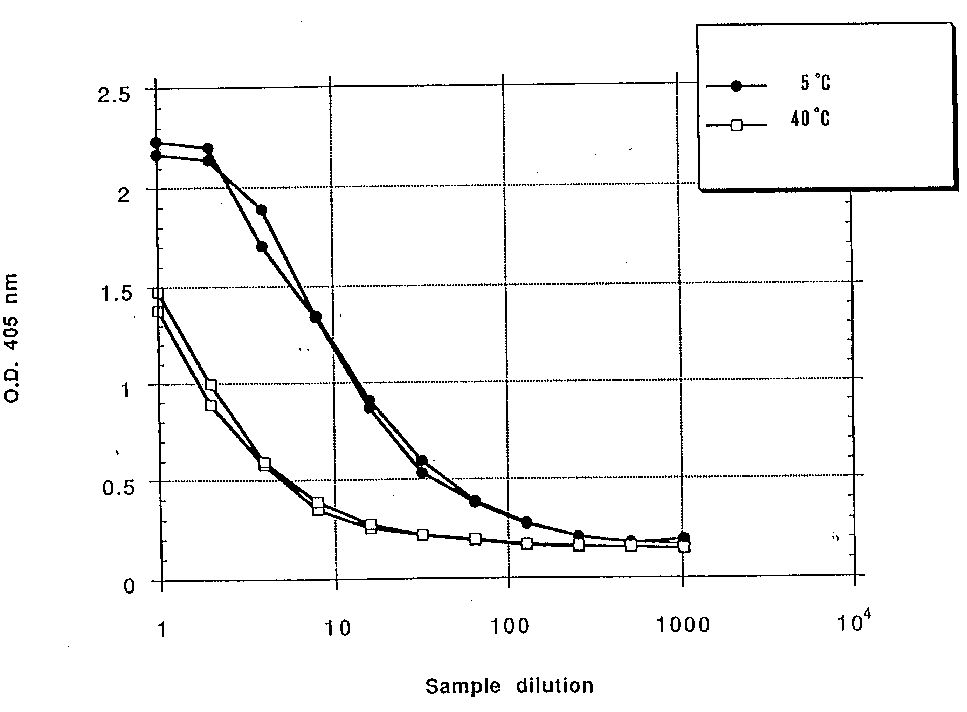

At a diluation factor 10 where the optical density /dilution factor curves in figures 6 to 8 is reasonable linear the optical density was for tetanus toxoid 1.2 at 5°C and only 0.30 at 40°C after a storage for 1 week. (See Figure 6, 2 batches).

This means that a reduction of tetanus toxoid activity has taken place from 4 to 1 ( = 75%).

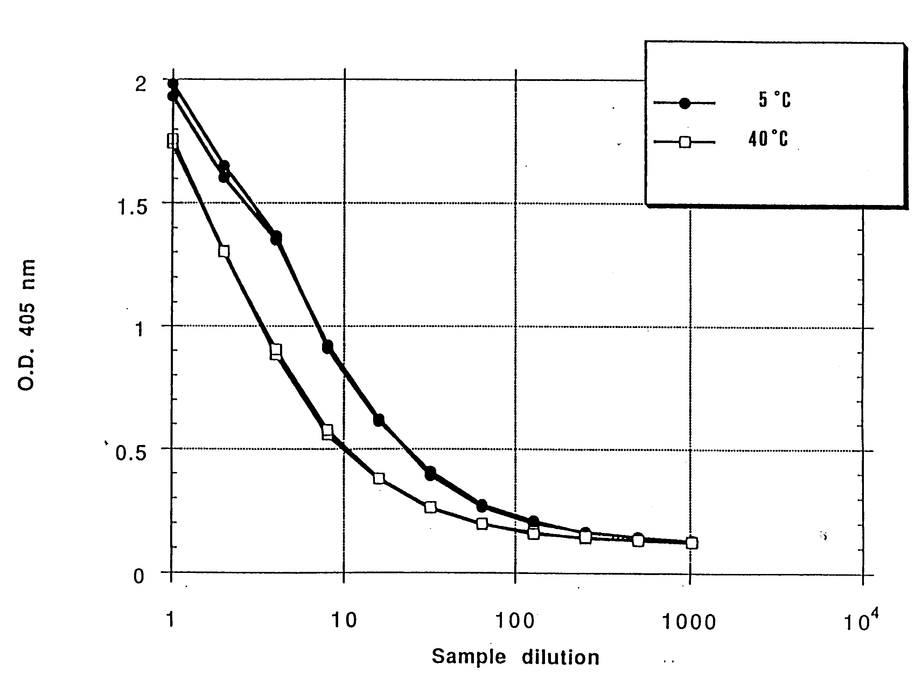

With the tetanus toxoid pamoate salt the optical density under comparable circumstances was reduced from 0.75 to 0.50 (see Figure 7, duplicates of one batch), which means a reduction of tetanus toxoid activity from 3 to 2 (= 4 to 2.66) = 33%. The samples were not irradiated.

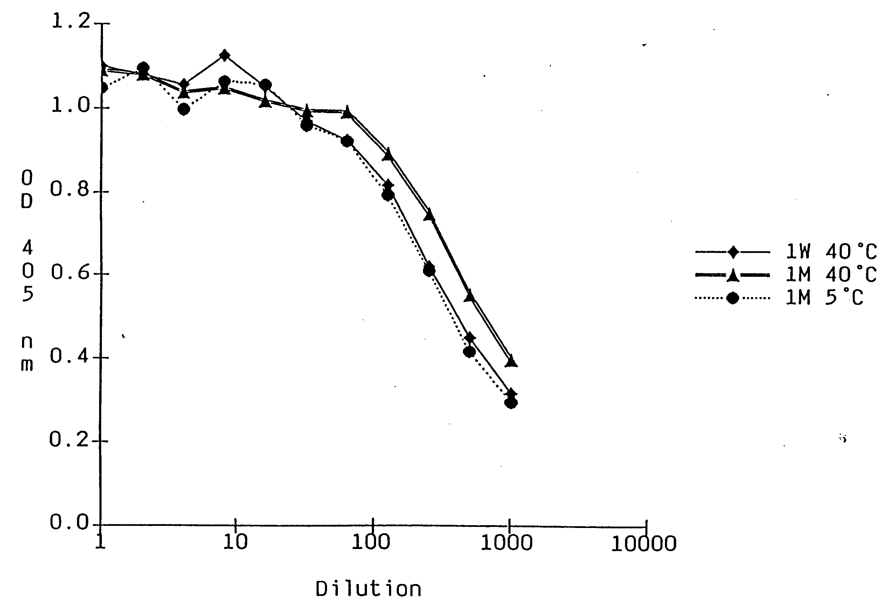

With sodium ascorbate (Example 4a) at a dilution factor 10 there was no tetanus toxoid reduction at all (see Figure 8, one batch was stored at 5° (1 month) and at 40°C (during 1 week and 1 month)).

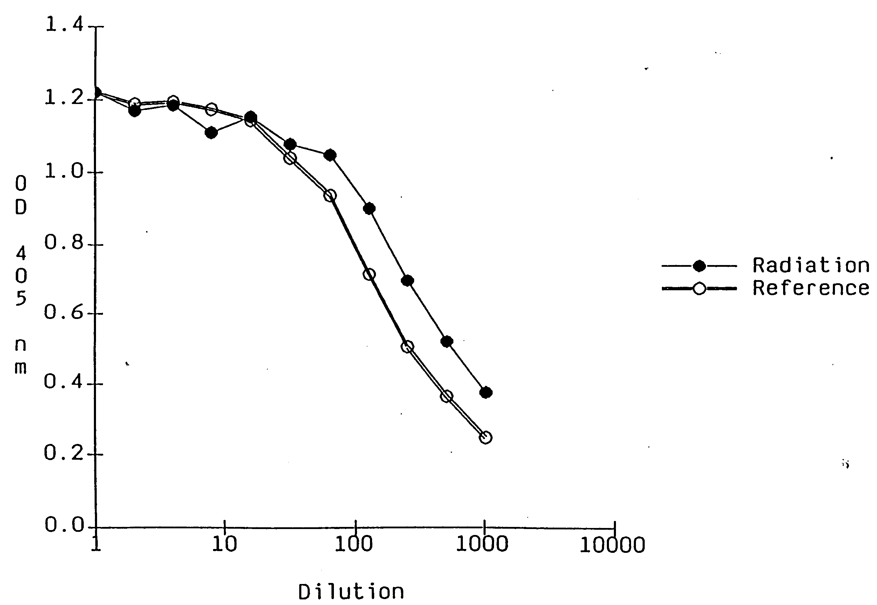

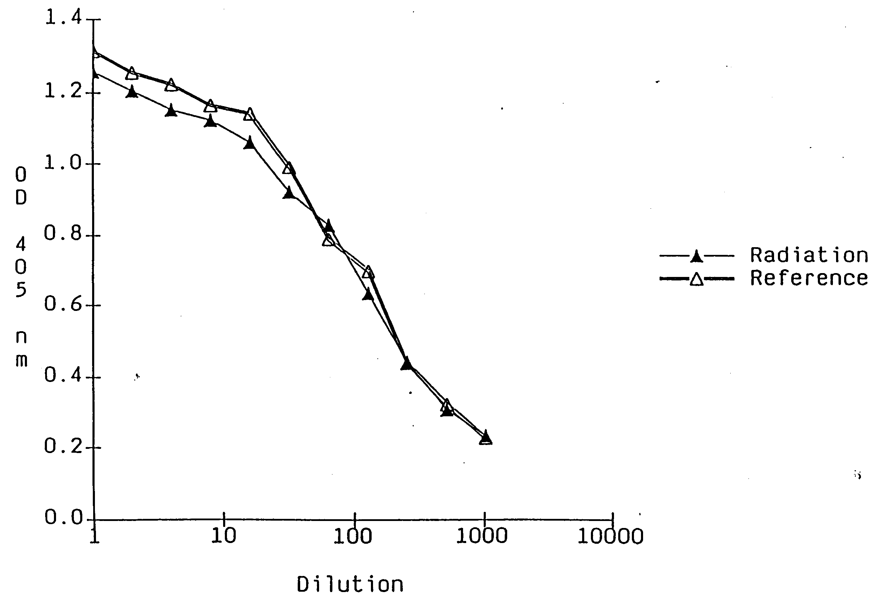

The same was ascertained when the samples were irradiated (Examples 4b and 4c and Figures 9 and 10).

## Claims

1. A pharmaceutical composition comprising as the active agent a peptide in association with embonic acid or ascorbic acid or a salt thereof, excluding
1) ACTH in association with embonic acid or acorbic acid or a salt thereof and
2) a LHRH active compound in association with ascorbic acid or a salt thereof,
in a matrix of a polylactide, having a lactide / glycolide molar ratio of from 100:0 to 40:60, a Mw of from 10.000 to 200.000 and a polydispersity of from 1.7 to 3.0.

2. A pharmaceutical composition according to claim 1 showing, after manufacture thereof an increase of at most 0.5% in active agent degradation products, resulting in a total degradation product content of at most 2.5%, the composition showing in an aqueous medium an active agent release which is continuous during a period of at least 2 weeks and amounts within the first 24 hours to at most 15% of weight of active agent released.

3. CH₃-CO-D-2-Nal-DPhe(p-CI)-DTrp-Ser-Tyr-DLys(CH₂-CHOH-CH₂OH)-Leu-Lys(CH₂-CHOH-CH₂OH)-Pro-DAla-NH₂ as an embonic acid or ascorbic salt.

4. A pharmaceutical composition according to claim 1 comprising a compound of claim 3.

5. A method of stabilizing a water soluble pharmacologically active agent which is the peptide CH₃-CO-D-2-Nal-DPhe(p-CI)-DTrp-Ser-Tyr-DLys(CH₂-CHOH-CH₂OH)-Leu-Lys(CH₂-CHOH-CH₂OH)-Pro-DAla-NH₂ in a matrix of a polylactide, having a lactide/glycolide molar ratio of from 100:0 to 40:60, a Mw of from 10.000 to 200.000 and a polydispersity of from 1.7 to 3.0 which comprises bringing the active agent in association with embonic acid or ascorbic acid or a salt thereof.

6. Use of embonic acid or ascorbic acid or a salt thereof to stabilize the peptide CH₃-CO-D-2-Nal-DPhe(p-CI)-DTrp-Ser-Tyr-DLys(CH₂-CHOH-CH₂OH)-Leu-Lys(CH₂-CHOH-CH₂OH)-Pro-DAla-NH₂ in a matrix of a polylactide, having a lactide/glycolide molar ratio of from 100:0 to 40:60, a Mw of from 10.000 to 200.000 and a polydispersity of from 1.7 to 3.0.

7. A pharmaceutical composition according to claim 1 comprising as an active agent tetanus toxoid.

8. A composition according to claim 7 showing when stored during a week at a temperature of 40°C compared with a composition stored during a week at 5°C having an activity reduction of at most 33%.

9. Tetanus toxoid pamoate salt.

10. Tetanus toxoid ascorbate salt.

11. A method of stabilizing a tetanus toxoid in a matrix of a polylactide, having a lactide/glycolide molar ratio of from 100:0 to 40:60, a Mw of from 10.000 to 200.000 and a polydispersity of from 1.7 to 3.0 which comprises bringing the tetanus toxoid in association with embonic acid or ascorbic acid or a salt therof.

12. Use of embonic acid or ascorbic acid or a salt thereof to stabilize a tetanus toxoid in a matrix of a polylactide, having a lactide/glycolide molar ratio of from 100:0 to 40:60, a Mw of from 10.000 to 200.000 and a polydispersity of from 1.7 to 3.0.
